# EUROPÄISCHE PATENTSCHRIFT

(11) **EP 1 036 055 B1**
(45) Veröffentlichungstag und Bekanntmachung des Hinweises auf die Patenterteilung: **27.03.2002**
(21) Anmeldenummer: 98963492.8
(22) Anmeldetag: 21.11.1998
(51) Int. Cl.: C07C 51/00

(54) **VERFAHREN ZUR HERSTELLUNG VON CHLORCARBONSÄURECHLORIDEN**
METHOD FOR PRODUCING CHLOROCARBOXYLIC ACID CHLORIDES
PROCEDE DE PREPARATION DE CHLORURES D'ACIDE CHLOROCARBOXYLIQUE

(30) Priorität: 04.12.1997 DE 19753773
(43) Veröffentlichungstag der Anmeldung: 20.09.2000
(73) Patentinhaber: BASF AKTIENGESELLSCHAFT, 67056 Ludwigshafen (DE)
(72) Erfinder: WEYER, Hans-Jürgen, D-67240 Bobenheim-Roxheim (DE); STAMM, Armin, D-55128 Mainz (DE); WEBER, Theodor, D-67063 Ludwigshafen (DE); HENKELMANN, Jochem, D-68165 Mannheim (DE)
(86) Internationale Anmeldenummer: EP9807500
(87) Internationale Veröffentlichungsnummer: WO9929648

(56) Entgegenhaltungen:
- EP-A- 0 253 214
- EP-A- 0 413 264
- US-A- 2 778 852
- US-A- 4 129 595
- CHEMICAL ABSTRACTS, vol. 84, no. 21, 24. Mai 1976 Columbus, Ohio, US; abstract no. 150214w, Seite 510; XP002099244 & JP 51 001410 A (NIPPON POLYURETHANE INDUSTRY CO., LTD.)

## Beschreibung

Die vorliegende Erfindung betrifft ein neues Verfahren zur Herstellung von Chlorcarbonsäurechloriden durch Umsetzung von Lactonen mit Chlorierungsmitteln in Gegenwart von Harnstoffverbindungen.

Chlorcarbonsäurechloride sind bekanntermaßen wertvolle Zwischenprodukte für organische Synthesen, insbesondere von Pflanzenschutzmitteln und Arzneimitteln.

Aus der DE-A 36 24 258 ist die Herstellung von Chlorcarbonsäurechloriden durch Umsetzung der entsprechenden Lactone mit Phosgen in Gegenwart quartärer Ammoniumsalze als Katalysatoren bekannt. Obwohl man nach diesem Verfahren gute Ausbeuten erzielt, läßt es in verfahrenstechnischer Hinsicht zu wünschen übrig, weil die quartären Ammoniumsalze sowohl in den Lactonen als auch in den Chlorcarbonsäurechloriden unlöslich sind, weswegen sich die Aufarbeitung des Reaktionsgemisches und besonders auch eine kontinuierliche Verfahrensführung erschweren.

Die EP-A 413 264 und die EP-A 435 714 beschreibt ein Verfahren zur Herstellung von Chlorcarbonsäurechloriden durch Chlorierung von Lactonen mit Phosgen in Gegenwart von Phosphinoxiden, bei dem phosphorhaltige Destillationsrückstände anfallen, deren Entsorgung durch die Bildung von Phosphorsäure bei der Verbrennung der Rückstände erschwert wird.
Weiterhin ist aus der US-A 341 8 365 ein Verfahren zur Herstellung von Chlorcarbonsäurechloriden aus Lactonen mit Thionylchlorid bekannt, mit dem jedoch nur unbefriedigende Ausbeuten erzielt werden.

Der Erfindung lag die Aufgabe zugrunde, ein Verfahren zur Herstellung von Chlorcarbonsäurechloriden durch Umsetzung von Lactonen mit Chlorierungsmitteln bereit zustellen, das die Nachteile der bekannten Verfahren vermeidet.

Die Aufgabe wird erfindungsgemäß gelöst durch ein Verfahren zur Herstellung von Chlorcarbonsäurechloriden der Formel I

R¹-CHCl-Y-COCl (I),

in der
- R¹: Wasserstoff oder gegebenenfalls mit C₁- bis C₄-Alkoxy, C₂- bis C₄-Acyl, C₂- bis C₄-Acyloxy, C₂- bis C₈-Dialkylamino, Halogen, Nitro und/oder Cyano ein- bis dreifach substituiertes C₁- bis C₂₀-Alkyl, C₂- bis C₁₂-Alkenyl, C₂- bis C₁₂-Alkinyl oder gegebenenfalls mit C₁- bis C₄-Alkyl, C₁- bis C₄-Alkoxy, C₂- bis C₄-Acyl, C₂- bis C₄-Acyloxy, C₂- bis C₈-Dialkylamino, Halogen, Nitro und/oder Cyano ein- bis dreifach substituiertes C₃- bis C₁₂-Cycloalkyl, C₄- bis C₁₂-Alkylcycloalkyl-, C₄- bis C₁₂-Cycloalkylalkyl, Heterocycloalkyl, C₅- bis C₂₀-Heterocycloalkyl-alkyl, C₆- bis C₁₄-Aryl, C₇- bis C₂₀-Arylalkyl oder C₇- bis C₂₀-Alkylaryl bedeuten und
- Y: eine, gegebenenfalls C₁- bis C₁₂-Alkyl, C₁- bis C₈-Alkoxy, C₂-C₄-Acyl, C₂-C₄-Acyloxy, C₂-C₈-Dialkylamino, Halogen, Nitro und Cyano ein- bis dreifach substituierte, gesättigte oder ein- oder mehrfach olefinisch ungesättigte C₂-C₈-Alkylenkette, die durch eine Ether-, Thioether-, tertiäre Amino-, Keto-, Lacton, N-alkylsubstituierte Lactam- oder Sulfongruppe unterbrochen sein kann, bedeutet,
durch Umsetzung des Lactons der Formel II 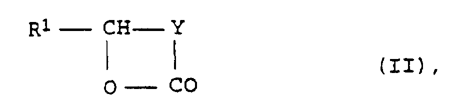 in der R¹ und Y die vorstehend genannte Bedeutung zukommt, mit einem Chlorierungsmittel in Gegenwart einer Harnstoffverbindung der Formel III

R²R³N-CX-NR⁴R⁵ (III),

in der die Reste R², R³, R⁴ und R⁵ gleich oder verschieden sein können und unabhängig voneinander die für R¹ gegebene Bedeutung mit Ausnahme von Wasserstoff haben oder in der einer der Reste R² oder R³ gemeinsam mit einem der Reste R⁴ oder R⁵ eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und Cyano ein- bis dreifach substituierte gesättigte oder ein- oder mehrfach olefinisch ungesättigte Kohlenstoffkette mit 2 bis 8 C-Atomen, die eine Ether-, Thioether-, tertiäre Amino-, Keto-, Lacton-, alkylsubstituierte Lactam-, Sulfon- oder Diketogruppe enthalten kann, oder eine, gegebenenfalls mit C₁-C₄-Alkyl, C₁-C₄-AlkoxyPhenoxy, C₂-C₄-Acyl, C₂-C₄-Acyloxy, C₂-C₈-Dialkylamino, Halogen, Nitro und/oder Cyano ein- bis dreifach substituierte Cycloalkylen mit 5 bis 12 C-Atomen, Heterocycloalkylengruppe mit 4 bis 11 C-Atomen, Arylengruppe mit 6 bis 12 C-Atomen oder Heteroarylengruppe mit 3 bis 11 C-Atomen bedeuten kann und in der X für ein Sauerstoff- oder Schwefelatom steht und/oder einer Harnstoffverbindung der allgemeinen Formel IV 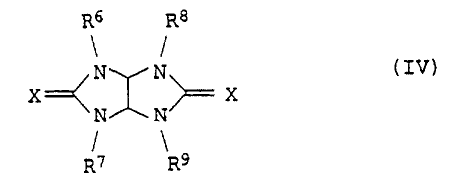 worin die angegebene Bedeutung hat, R⁶, R⁷, R⁸ und R⁹ gleich oder verschieden sein können und unabhängig voneinander die für R¹ gegebene Bedeutung mit Ausnahme von Wasserstoff haben
und/oder
einer Verbindung der allgemeinen Formel (V), 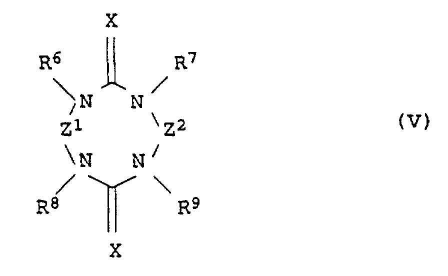 worin X, R⁶ bis R⁹ die oben angegebenen Bedeutungen und Z¹, Z², die gleich oder verschieden sein können, eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und/oder Cyano ein- bis dreifach substituierte Methylen-, Ethylen- oder Vinylengruppe bedeuten.

Y steht für eine C₂-C₈-Alkylenkette, die durch Heteroatome wie Sauerstoff oder Schwefel oder durch gegenüber dem Chlorierungsmittel unter den Reaktionsbedingungen inerte Gruppen wie eine tertiäre Amino-, Keto-, Lacton-, N-alkylsubstituierte Lactam- oder Sulfongruppe unterbrochen sein. Y kann ferner mit unter den Reaktionsbedingungen gegenüber dem Chlorierungsmittel inerten Gruppen wie C₁-C₄-Alkyl, C₁-C₄-Alkoxy, C₂-C₄-Acyl, C₂-C₄-Alcyloxy, C₂-C₈-Dialkylamino, Halogen, Nitro und Cyano ein- bis dreifach substituiert sein. Bevorzugt enthält Y nur C-Atome als Kettenbausteine und keine weiteren Substituenten. Besonders bevorzugt steht Y für C₂-C₄-Alkylen wie Ethylen, Propylen und Butylen, insbesondere für Ethylen und Propylen.

Die organischen Substituenten R¹ bis R⁹ in den Verbindungen I, II, III, IV und V haben unabhängig voneinander folgende Bedeutungen:
- C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl, insbesondere C₁- bis C₄-Alkyl wie Methyl, Ethyl, n-Propyl, iso-Propyl, n-Butyl, iso-Butyl, sec.-Butyl und tert.-Butyl,
- C₁-C₁₂-Alkenyl, bevorzugt C₂-C₈-Alkenyl, wie Vinyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-propenyl, 1-Ethyl-1-propenyl, 1-Ethyl-2-propenyl, 1-Hexenyl, 2-Hexenyl, 3-Hexenyl, 4-Hexenyl, 5-Hexenyl, besonders bevorzugt Vinyl, 2-Propenyl und 1-Butenyl,
- C₁- bis C₁₂-Alkinyl, bevorzugt C₂-C₈-Alkinyl, wie Ethinyl, 1-Propinyl, 2-Propinyl, 1-Butinyl, 3-Butinyl, 1-Methyl-2-propinyl, 1-Pentinyl, 2-Pentinyl, 3-Pentinyl, 1-Methyl-2-butinyl, 1-Methyl-3-butinyl,2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, besonders bevorzugt Ethinyl, 1-Propinyl und 1-Butinyl.

Die genannten Gruppen können mit C₁-C₄-Alkoxy, C₂-C₄-Acyl, C₂-C₄-Acyloxy, C₂-C₈-Dialkylamino, Halogen, Nitro und/oder Cyano substituiert sein. Bevorzugt sind substituierte Alkylreste, besonders bevorzugt mit Halogen oder Cyano, substituierte Alkylreste wie Cyanmethyl, Chlormethyl.

R¹ bis R⁹ stehen weiterhin für:
- C₃- bis C₁₂-Cycloalkyl, bevorzugt C₅- bis C₈-Cycloalkyl wie Cyclopentyl, Cyclohexyl, Cycloheptyl und Cyclooctyl, besonders bevorzugt Cyclopentyl und Cyclohexyl,
- C₄- bis C₁₂-Alkylcycloalkyl, bevorzugt C₅- bis C₁₀-Alkylcycloalkyl, besonders bevorzugt C₅- bis C₈-Alkylcycloalkyl,
- C₄- bis C₁₂-Cycloalkyl-alkyl, bevorzugt C₅- bis C₁₀-Cycloalkyl-alkyl, besonders bevorzugt C₅- bis C₈-Cycloalkyl-alkyl,
- C₅- bis C₂₀-Alkyl-cycloalkyl-alkyl, bevorzugt C₆- bis C₁₆-Alkyl-cycloalkyl-alkyl, besonders bevorzugt C₇- bis C₁₂-Alkylcycloalkyl-alkyl,
- Heterocycloalkyl- wie ein 5- oder 6-gliedriger Ring oder einem oder zwei O-, N- und/oder S-Atomen im Ring, der aromatisch oder nichtaromatisch sein kann, wie 2- oder 3-Furyl, 2- oder 3-Thienyl, 2- oder 3-Pyrrolyl, 2- oder 4-Imidazolyl, 2- oder 3-Oxazolyl, 2- oder 3-Oxazolyl, 2- oder 3-Thiazolyl, Pyridinyl, Morpholyl, Thiomorpholyl und Pyrazolyl,
- C₆- bis C₁₄-Aryl wie Phenyl, 1-Naphthyl und 2-Naphthyl, bevorzugt Phenyl,
- C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2 -Ethylphenyl, 3-Ethylphenyl und 4-Ethylphenyl,
- C₇ bis C₂₀-Arylalkyl, bevorzugt C₇- bis C₁₆-Aralkyl, bevorzugt C₇- bis C₁₂-Phenalkyl wie Benzyl, 1-Phenylethyl, 2-Phenylethyl, 3-Phenylpropyl, 1-Phenylbutyl, 2-Phenylbutyl, 3-Phenylbutyl, 4-Phenylbutyl, besonders bevorzugt Benzyl, 1-Phenylethyl und 2-Phenylethyl.

Die Reste R² oder R³ können weiterhin gemeinsam mit einem der Reste R⁴ oder R⁵ eine Cycloalkylengruppe mit 5 bis 12 C-Atomen bedeuten, die über vicinale C-Atome oder in 1,3-Stellung gebunden sein kann. Beispiele sind 1,2- oder 1,3-Cyclopentylen, 1,2- oder 1,3-Cyclohexylen und 1,2- oder 1,3-Cycloheptylen. Die Cycloalkylengruppe kann an Stelle von einem oder mehreren C-Atomen auch ein oder mehrere Heteroatome wie Stickstoff, Sauerstoff oder Schwefel enthalten. Die Reste R² oder R³ können ferner gemeinsam mit einem der Reste R⁴ oder R⁵ eine (divalente) Arylengruppe mit 6 bis 12 C-Atomen bedeuten, die über vicinale C-Atome gebunden ist. Beispiele sind ortho-Phenylen, 1,2- oder 2,3-Naphthylen, und 1,2 oder 2,3-Anthracenylen, bevorzugt ortho-Phenylen. Die Arylengruppe kann an Stelle von einem oder mehreren C-Atomen auch ein oder mehrere Heteroatome wie Stickstoff enthalten. Beispiele sind 2,3- oder 3,4-Pyrrolylen, 2,3- oder 3,4-Pyridinylen, 2,3-, 3,4-, 5,6- oder 6,7-Chinolinylen- oder 2,3-, 5,6- oder 6,7-Chinoxalinylen.

Als Harnstoffverbindungen der Formel III werden bevorzugt jene verwendet, die bei Reaktionsbedingungen flüssig sind, besonders bevorzugt N,N'-Dimethylethylenharnstoff, N,N'-Dimethylpropylenharnstoff, N,N'-Tetrabutylharnstoff, N,N'-Tetramethylthioharnstoff, N-Chlormethyl-N'-cyanomethylpropylenharnstoff, N-Methyl-N'-ethylpropylenharnstoff, 1,3-Dimethyl-1,3-dihydrobenzimidazol-2-on, 1-Methyl-3-phenylimidazolidin-2,4,5-trion, 1,3,4,6-Tetramethyl-1,3,4,6-tetrahydroimidazo[4,5 D]imidazol-2,5 dion und 4-Methoxy-1-methyl-3-phenyl-imidazlidim-2-thion.

Die genannten Harnstoffverbindungen können als solche oder in Form ihrer Salze mit Halogenwasserstoffsäuren, beispielsweise als Hydrochloride, oder in Form ihrer durch Umsetzung mit Phosgen erhältlichen Salze (Vilsmeier Salze) eingesetzt werden, wobei die Hydrochloride bevorzugt sind.

Die Harnstoffverbindungen der Formel III, IV und V sind beispielsweise nach den in Houben-Weyl, Methoden der organischen Chemie, (1983) Band E 4, Seiten 335 - 391 und Seiten 484 - 505 beschriebenen Umsetzungen zugänglich.

Die Harnstoffverbindungen der Formel III, IV und/oder V werden in einem Molverhältnis zum Lacton II von 0,001:1 bis 0,2:1, bevorzugt 0,002:1 bis 0,1:1, besonders bevorzugt 0,005:1 bis 0,05:1 eingesetzt.

Als Ausgangsverbindungen II sind im Hinblick auf die Bedeutung der Verfahrensprodukte I vor allem die unsubstituierten Lactone, also das Butyrolacton, das Valerolacton und das Caprolacton zu nennen.

Werden flüssige, niedrig schmelzende Lactone eingesetzt, so kann die Reaktion in aller Regel ohne Lösungsmittel durchgeführt werden. Ansonsten empfiehlt es sich, die Umsetzung in einem inerten Lösungsmittel vorzunehmen. Als Lösungsmittel eignen sich hochsiedende Kohlenwasserstoffe wie Cumol, Toluol, Xylol, Benzol oder aromatische Chlorverbindungen wie Chlorbenzol oder Dichlorbenzol oder auch bevorzugt das Verfahrensprodukt in Mengen von bis zu 50 Gew.-%, bezogen auf das Lacton II.

Als Chlorierungsmittel können an sich bekannte Reagenzien wie Phosgen, Thionylchlorid, Oxalylchlorid, und Phosphortrichlorid eingesetzt werden, wobei die Verwendung von Phosgen bevorzugt ist. Das Molverhältnis des Chlorierungsmittels zum Lacton II beträgt von 0,5:1 bis 50:1, bevorzugt 1:1 bis 2:1 besonders bevorzugt 1:1 bis 1,5:1 und insbesondere bevorzugt 1:1 bis 1,2:1

Häufig ist die zusätzliche Mitverwendung von Chlorwasserstoff vorteilhaft, und zwar in Mengen von 5 bis 100 mol%, vorzugsweise 20 bis 40 mol%, des Lactons II.
Das Lacton II wird in dem erfindungsgemäßen Verfahren in Gegenwart der Harnstoffverbindung III, IV oder V bei Temperaturen von -20 bis 180°C, bevorzugt 0 bis 120°C, besonders bevorzugt 40 bis 100°C und Drücken von 0,01 bis 50 bar, bevorzugt 0,5 bis 5 bar, besonders bevorzugt 0,9 bis 1 bar, insbesondere bevorzugt bei Atmosphärendruck, umgesetzt.

Wird die Reaktion diskontinuierlich durchgeführt, so empfiehlt es sich, eine Lösung aus dem Lacton und der Harnstoffverbindung sowie gegebenenfalls dem Lösungsmittel herzustellen und das Chlorierungsmittel mit der Geschwindigkeit in diese Lösung einzuleiten, mit der es reagiert. Auf diese Weise kommt man mit 1 bis 3 Mol Chlorierungsmittel pro Mol Lacton II aus. Der Überschuß an Chlorierungsmittel kann in üblicher Art und Weise in die Reaktionsgefäße zurückgeführt werden.

Die Vorteile des erfindungsgemäßen Verfahrens treten bei kontinuierlicher Fahrweise besonders hervor, sei es unter Verwendung einer Rührkesselkaskade, einer im Gegenstrom betriebenen Reaktionskolonne, einem Schlaufenreaktor, einem Reaktionsrohr oder einer Kombination der vorstehend genannten Reaktoren.

Die Aufarbeitung des Reaktionsgemisches erfolgt in an sich bekannter Weise, und zwar im allgemeinen durch Destillation, wobei dieser bei Verwendung von Phosgen als Chlorierungsmittel gewünschtenfalls die Entfernung von überschüssigem Phosgen vorausgeht.

Vorteilhaft an dem erfindungsgemäßen Verfahren ist, daß die Umsetzung unter vergleichsweise milden Bedingungen mit geringen Mengen der Harnstoffverbindung vollständig und selektiv verläuft. Die Aufarbeitung des Reaktionsproduktes kann mit üblichen Ausarbeitungsmethoden ohne den Anfall phosphorhaltiger Rückstände erfolgen und eine kontinuierliche Durchführung des Verfahrens ist möglich.

Die Erfindung wird durch die nachstehenden Beispiele näher erläutert.

### Beispiele

### Beispiele 1 bis 4

In eine Mischung aus 3 mol gamma-Butyrolacton und 0,02 mol des Harnstoffverbindung III (R², R⁴=CH₃, Z variabel) wurden im Lauf von 7 Stunden bei 140°C b mol Phosgen und c mol Chlorwasserstoff eingeleitet.
Nach beendeter Umsetzung wurde das Reaktionsgemisch bei etwa 80°C durch Durchleiten von Stickstoff von überschüssigem Phosgen und Chlorwasserstoff befreit und gaschromatographisch analysiert. Die Einzelheiten der Beispiele 1 bis 4 sowie deren Ergebnisse sind der folgenden Tabelle 1 zu entnehmen.

### Beispiel 5

In die Mischung aus 3 mol Lacton II und 0,02 mol Dimethylpropylenharnstoff wurde im Laufe von 7 Stunden bei 140°C b mol Thionylchlorid zugetropft und c mol Chlorwasserstoffgas eingeleitet. Nach beendeter Umsetzung wurde das Reaktionsgemisch bei etwa 80°C durch Durchleiten von Stickstoff von überschüssigem Chlorwasserstoff befreit und gaschromatographisch analysiert. Die Einzelheiten des Beispiels 4 sowie dessen Ergebnis sind der folgenden Tabelle 1 zu entnehmen.

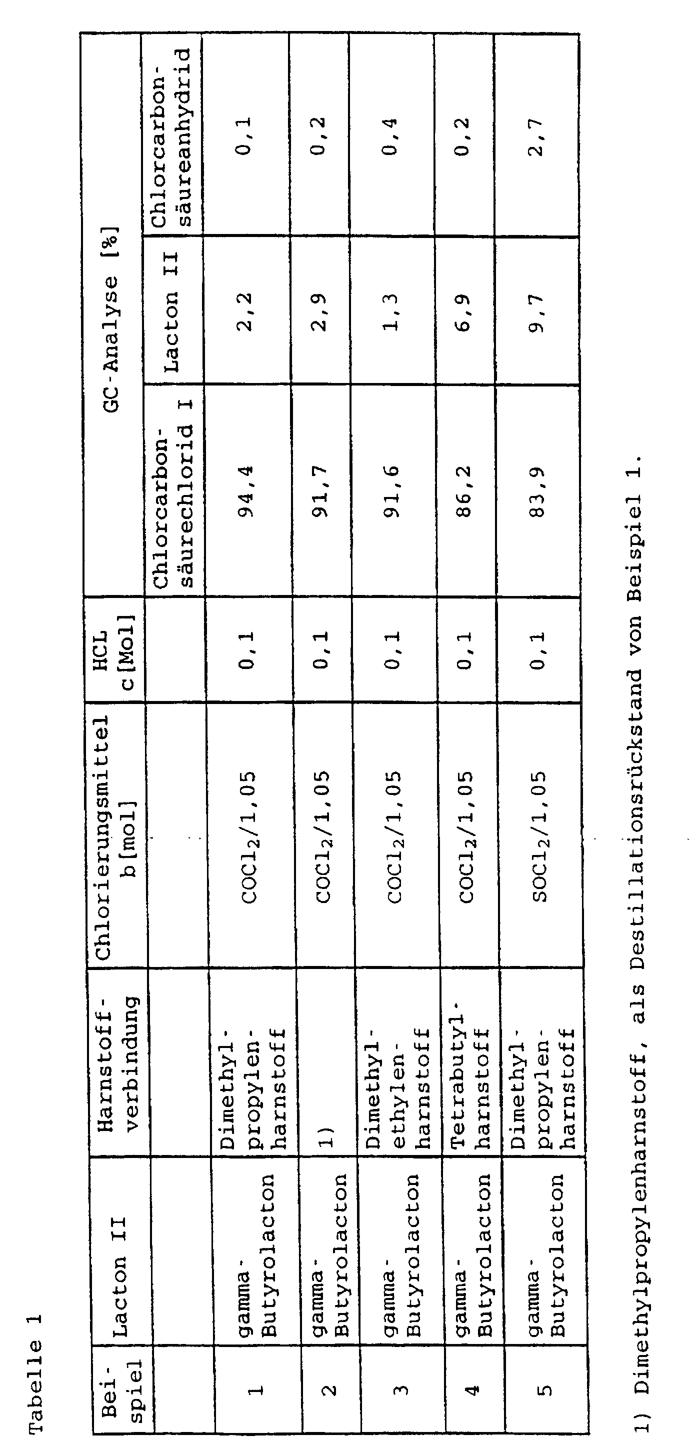

## Patentansprüche

1. Verfahren zur Herstellung von Chlorcarbonsäurechloriden der Formel I
R¹-CHCl-Y-COCl (I),
in der
R¹ Wasserstoff oder gegebenenfalls mit C₁- bis C₄-Alkoxy, C₂- bis C₄-Acyl, C₂- bis C₄-Acyloxy, C₂- bis C₈-Dialkylamino, Halogen, Nitro und/oder Cyano ein- bis dreifach substituiertes C₁- bis C₂₀-Alkyl, C₂- bis C₁₂-Alkenyl, C₂- bis C₁₂-Alkinyl oder gegebenenfalls mit C₁- bis C₄-Alkyl, C₁- bis C₄-Alkoxy, C₂- bis C₄-Acyl, C₂- bis C₄-Acyloxy, C₂- bis C₈-Dialkylamino, Halogen, Nitro und/oder Cyano ein- bis dreifach substituiertes C₃- bis C₁₂-Cycloalkyl, C₄- bis C₁₂-Alkylcycloalkyl-, C₄- bis C₁₂-Cycloalkylalkyl, Heterocycloalkyl, C₅- bis C₂₀-Heterocycloalkyl-alkyl, C₆- bis C₁₄-Aryl, C₇- bis C₂₀-Arylalkyl oder C₇- bis C₂₀-Alkylaryl bedeuten und
Y eine, gegebenenfalls C₁- bis C₄-Alkyl, C₁- bis C₄-Alkoxy, C₂-C₄-Acyl, C₂-C₄-Acyloxy, C₂-C₈-Dialkylamino, Halogen, Nitro und Cyano ein- bis dreifach substituierte gesättigte oder ein- oder mehrfach olefinisch ungesättigte C₂-C₈-Alkylenkette, die durch eine Ether-, Thioether-, tertiäre Amino-, Keto-, Lacton, N-alkylsubstituierte Lactam- oder Sulfongruppe unterbrochen sein kann, bedeutet,
durch Umsetzung des Lactons der Formel II 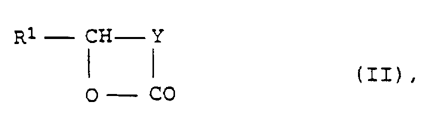 in der R¹ und Y die vorstehend genannte Bedeutung zukommt, mit einem Chlorierungsmittel in Gegenwart einer Harnstoffverbindung der Formel III
R²R³N-CX-NR⁴R⁵ (III),
in der die Reste R² R³, R⁴ und R⁵ gleich oder verschieden sein können und unabhängig voneinander die für R¹ gegebene Bedeutung mit Ausnahme von Wasserstoff haben oder in der einer der Reste R² oder R³ gemeinsam mit einem der Reste R⁴ oder R⁵ eine, gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und Cyano ein- bis dreifach substituierte gesättigte oder ein- oder mehrfach olefinisch ungesättigte Kohlenstoffkette mit 2 bis 8 C-Atomen, die eine Ether-, Thioether-, tertiäre Amino-, Keto-, Lacton-, alkylsubstituierte Lactam-, Sulfon- oder Diketogruppe enthalten kann, oder eine, gegebenenfalls mit C₁-C₄-Alkyl, C₁-C₄-Alkoxy- Phenoxy, C₂-C₄-Acyl, C₂-C₄-Acyloxy, C₂-C₈-Dialkylamino, Halogen, Nitro und/oder Cyano ein- bis dreifach substituierte Cycloalkylen mit 5 bis 12 C-Atomen, Heterocycloalkylengruppe mit 4 bis 11 C-Atomen, Arylengruppe mit 6 bis 12 C-Atomen oder Heteroarylengruppe mit 3 bis 11 C-Atomen bedeutet und in der X für ein Sauerstoff- oder Schwefelatom steht und/oder einer Harnstoffverbindung der allgemeinen Formel IV 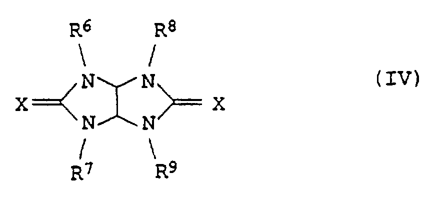 worin X die angegebene Bedeutung hat, R⁶, R⁷, R⁸ und R⁹ gleich oder verschieden sein können und unabhängig voneinander die für R¹ gegebene Bedeutung mit Ausnahme von Wasserstoff haben
und/oder
einer Verbindung der allgemeinen Formel (V), 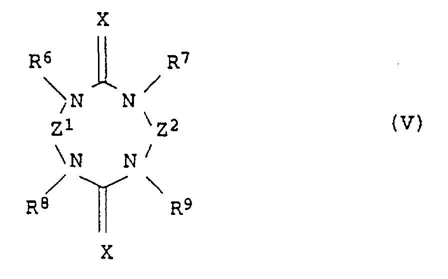 worin X, R⁶ bis R⁹ die oben angegebenen Bedeutungen und Z¹, Z², die gleich oder verschieden sein können, eine gegebenenfalls mit C₁-C₄-Alkyl, C₁-C₄-Alkoxy, C₂-C₄-Acyl, C₂-C₄-Acyloxy, Phenoxy, C₂-C₈-Dialkylamino, Halogen, Nitro und/oder Cyano ein- bis dreifach substituierte Methylen-, Ethylen- oder Vinylengruppe bedeuten.

2. Verfahren zur Herstellung von Chlorcarbonsäurechloriden nach Anspruch 1, **dadurch gekennzeichnet, daß** man N,N'-Dimethylethylenharnstoff, N,N'-Dimethylpropylenharnstoff, N,N'-Tetrabutylharnstoff, N,N'-Tetramethylthioharnstoff, N-Chlormethyl-N'-cyanomethylpropylenharnstoff, N-Methyl-N'-ethylpropylenharnstoff, 1,3-Dimethyl-1,3-dihydrobenzimidazol-2-on, 1-Methyl-3-phenylimidazolidin-2,4,5-trion, 1,3,4,6-Tetramethyl-1,3,4,6-tetrahydro-imidazo[4,5-D]imidazol-2,5-dion und 4-Methoxy-1-methyl-3-phenylimidazolidin-2-thion einsetzt.

3. Verfahren zur Herstellung von Chlorcarbonsäurechloriden nach den Ansprüchen 1 oder 2, **dadurch gekennzeichnet, daß** die Harnstoffverbindung in einem Molverhältnis von 0,001:1 bis 0,2:1 zum Lacton II eingesetzt wird.

4. Verfahren zur Herstellung von Chlorcarbonsäurechloriden nach den Ansprüchen 1 bis 3, **dadurch gekennzeichnet, daß** als Chlorierungsmittel Phosgen, Thionylchlorid, Oxalylchlorid oder Phosphortrichlorid eingesetzt wird.

5. Verfahren zur Herstellung von Chlorcarbonsäurechloriden nach den Ansprüchen 1 bis 4, **dadurch gekennzeichnet, daß** das Chlorierungsmittel in einem Molverhältnis von 0,5:1 bis 50:1 zu dem Lacton II eingesetzt wird.

6. Verfahren zur Herstellung von Chlorcarbonsäurechloriden nach den Ansprüchen 1 bis 5, **dadurch gekennzeichnet, daß** man die Umsetzung in Gegenwart von Chlorwasserstoff vornimmt.

7. Verfahren zur Herstellung von Chlorcarbonsäurechloriden nach den Ansprüchen 1 bis 6, **dadurch gekennzeichnet, daß** man die Umsetzung bei Temperaturen von -20 bis 180°C durchführt.

## Claims

1. A process for preparing chlorocarbonyl chlorides of the formula I
R¹-CHCl-Y-COCl (I),
where
R¹ is hydrogen, or is C₁- to C₂₀-alkyl, C₂- to C₁₂-alkenyl, C₂- to C₁₂-alkynyl, each of which is unsubstituted or mono- to trisubstituted by C₁- to C₄-alkoxy, C₂- to C₄-acyl, C₂- to C₄-acyloxy, C₂- to C₈-dialkylamino, halogen, nitro and/or cyano, or is C₃- to C₁₂-cycloalkyl, C₄- to C₁₂-alkylcycloalkyl, C₄- to C₁₂-cycloalkylalkyl, heterocycloalkyl, C₅- to C₂₀-heterocycloalkylalkyl, C₆- to C₁₄-aryl, C₇- to C₂₀-arylalkyl or C₇- to C₂₀-alkylaryl, each of which is unsubstituted or mono- to trisubstituted by C₁- to C₄-alkyl, C₁- to C₄-alkoxy, C₂- to C₄-acyl, C₂- to C₄-acyloxy, C₂- to C₈-dialkylamino, halogen, nitro and/or cyano, and
Y is a saturated or mono- or polyolefinically unsaturated C₂-C₈-alkylene chain which may be interrupted by an ether, thioether, tertiary amino, keto, lactone, N-alkylsubstituted lactam or sulfone group and is unsubstituted or mono- to trisubstituted by C₁- to C₄-alkyl, C₁- to C₄-alkoxy, C₂-C₄-acyl, C₂-C₄-acyloxy, C₂-C₈-dialkylamino, halogen, nitro and cyano,
by reacting the lactone of the formula II 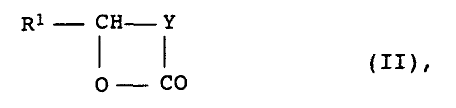 where R¹ and Y are each as defined above, with a chlorinating agent in the presence of a urea compound of the formula III
R²R³N-CX-NR⁴R⁵ (III),
where the radicals R^{2,} R³, R⁴ and R⁵ may be identical or different and, independently of the others, each has the meanings given for R¹ with the exception of hydrogen, or where one of the radicals R² or R³ together with one of the radicals R⁴ or R⁵ are a hydrocarbon chain having 2 to 8 carbon atoms which may contain an ether, thioether, tertiary amino, keto, lactone, alkyl-substituted lactam, sulfone or diketo group and which is saturated or mono- or polyolefinically unsaturated and is unsubstituted or mono- to trisubstituted by C₁-C₄-alkyl, C₁-C₄-alkoxy, C₂-C₄-acyl, C₂-C₄-acyloxy, phenoxy, C₂-C₈-dialkylamino, halogen, nitro and cyano, or are a cycloalkylene group having 5 to 12 carbon atoms, a heterocycloalkylene group having 4 to 11 carbon atoms, an arylene group having 6 to 12 carbon atoms or a heteroarylene group having 3 to 11 carbon atoms, each of which is unsubstituted or mono- to trisubstituted by C₁-C₄-alkyl, C₁-C₄-alkoxy, phenoxy, C₂-C₄-acyl, C₂-C₄-acyloxy, C₂-C₈-dialkylamino, halogen, nitro and/or cyano, and where X is an oxygen or sulfur atom, and/or a urea compound of the formula IV 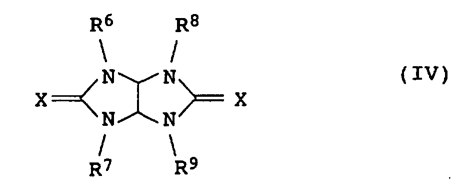 where X is as defined above, R⁶, R⁷, R⁸ and R⁹ may be identical or different and, independently of the others, each has the meanings given for R¹, with the exception of hydrogen,
and/or
a compound of the formula (V), 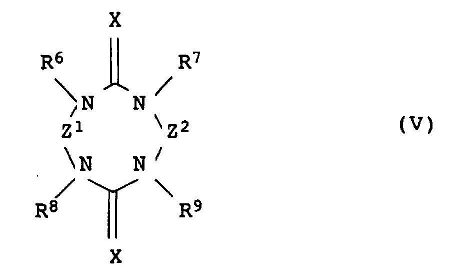 where X, R⁶ to R⁹ are each as defined above and Z¹, Z², which may be identical or different, are a methylene, ethylene or vinylene group which is unsubstituted or mono- to trisubstituted by C₁-C₄-alkyl, C₁-C₄-alkoxy, C₂-C₄-acyl, C₂-C₄-acyloxy, phenoxy, C₂-C₈-dialkylamino, halogen, nitro and/or cyano.

2. A process for preparing chlorocarbonyl chlorides as claimed in claim 1, wherein N,N-dimethylethyleneurea, N,N'-dimethylpropyleneurea, N,N'-tetrabutylurea, N,N'-tetramethylthiourea, N-chloromethyl-N'-cyanomethylpropyleneurea, N-methyl-N'-ethylpropyleneurea, 1,3-dimethyl-1,3-dihydrobenzimidazol-2-one, 1-methyl-3-phenylimidazolidine-2,4,5-trione, 1,3,4,6-tetramethyl-1,3,4,6-tetrahydroimidazo[4,5-D]imidazole-2,5-dione and 4-methoxy-1-methyl-3-phenylimidazolidine-2-thione are employed.

3. A process for preparing chlorocarbonyl chlorides as claimed in claim 1 or 2, wherein the urea compound is employed in a molar ratio of from 0.001:1 to 0.2:1 relative to the lactone II.

4. A process for preparing chlorocarbonyl chlorides as claimed in any of claims 1 to 3, wherein the chlorinating agent used is phosgene, thionyl chloride, oxalyl chloride or phosphorus trichloride.

5. A process for preparing chlorocarbonyl chlorides as claimed in any of claims 1 to 4, wherein the chlorinating agent is employed in a molar ratio of from 0.5:1 to 50:1 relative to the lactone II.

6. A process for preparing chlorocarbonyl chlorides as claimed in any of claims 1 to 5, wherein the reaction is carried out in the presence of hydrogen chloride.

7. A process for preparing chlorocarbonyl chlorides as claimed in any of claims 1 to 6, wherein the reaction is carried out at from -20 to 180°C.

## Revendications

1. Procédé de préparation de chlorures de chlorocarbonyle de formule I
R¹-CHCl-Y-COCl (I)
dans laquelle
R¹ est un atome d'hydrogène ou un groupe alkyle en C₁ à C₂₀, alcényle en C₂ à C₁₂, alcynyle en C₂ à C₁₂, éventuellement substitué une à trois fois par des substituants alcoxy en C₁ à C₄, acyle en C₂ à C₄, acyloxy en C₂ à C₄, dialkylamino en C₂ à C₈, halogéno, nitro et/ou cyano ; ou un groupe cycloalkyle en C₃ à C₁₂, alkylcycloalkyle en C₄ à C₁₂, cycloalkylalkyle en C₄ à C₁₂, hétérocycloalkyle, hétérocycloalkylalkyle en C₅ à C₂₀, aryle en C₆ à C₁₄, arylalkyle en C₇ à C₂₀ ou alkylaryle en C₇ à C₂₀, éventuellement une à trois fois substitué par des substituants alkyle en C₁ à C₄, alcoxy en C₁ à C₄, acyle en C₂ à C₄, acyloxy en C₂ à C₄, dialkylamino en C₂ à C₈, halogéno, nitro et/ou cyano, et
Y est une chaîne alkylène en C₂ à C₈, saturée ou portant une ou plusieurs insaturations oléfiniques, éventuellement substituée une à trois fois par des substituants alkyle en C₁ à C₄, alcoxy en C₁ à C₄, acyle en C₂ à C₄, acyloxy en C₂ à C₄, dialkylamino en C₂ à C₈, halogéno, nitro et cyano, chaîne alkylène qui peut être interrompue par un groupe lactame ou sulfone substitué par un substituant éther, thioéther, amino tertiaire, céto, lactone, N-alkyle,
par réaction de la lactone de formule II 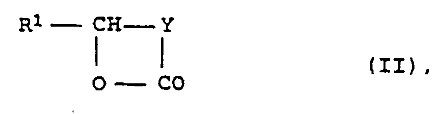 dans laquelle R¹ et Y ont les significations données ci-dessus, avec un agent de chloration en présence d'un dérivé de l'urée de formule III
R²R³N-CX-NR⁴R⁵ (III)
dans laquelle les radicaux R², R³, R⁴ et R⁵ peuvent être identiques ou différents, et chacun a, indépendamment des autres, la signification donnée pour R¹ à l'exception de l'hydrogène, ou encore dans laquelle l'un des radicaux R² ou R³, avec l'un des radicaux R⁴ ou R⁵, forme une chaîne carbonée ayant de 2 à 8 atomes de carbone, saturée ou portant une ou plusieurs insaturations oléfiniques, éventuellement substituée une à trois fois par des substituants alkyle en C₁-C₄, alcoxy en C₁-C₄, acyle en C₂-C₄, acyloxy en C₂-C₄, phénoxy, dialkylamino en C₂-C₈, halogéno, nitro et cyano, chaîne carbonée qui peut contenir un groupe éther, thioéther, amino tertiaire, céto, lactone, lactame alkylé, sulfone ou dicéto ; ou encore un radical cycloalkylène ayant de 5 à 12 atomes de carbone, éventuellement substitué une à trois fois par des substituants alkyle en C₁-C₄, alcoxy en C₁-C₄, phénoxy, acyle en C₂-C₄, acyloxy en C₂-C₄, dialkylamino C₂-C₈, halogéno, nitro et/ou cyano ; un groupe hétérocycloalkylène ayant de 4 à 11 atomes de carbone, un groupe arylène ayant de 6 à 12 atomes de carbone ou un groupe hétéroarylène ayant de 3 à 11 atomes de carbone ; et dans laquelle X est un atome d'oxygène ou de soufre ;
et/ou en présence d'un dérivé de l'urée de formule générale IV 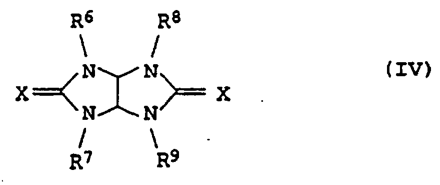 dans laquelle X a les significations données, R⁶, R⁷, R⁸ et R⁹ peuvent être identiques ou différents et ont chacun indépendamment des autres la signification de R¹ à l'exception de l'hydrogène, et/ou
d'un composé de formule générale V 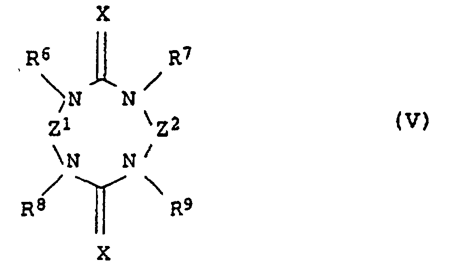 dans laquelle X, R⁶ à R⁹ ont les significations données ci-dessus, et Z¹, Z², qui peuvent être identiques ou différents, représentent chacun un groupe méthylène, éthylène ou vinylène éventuellement une à trois fois substitué par des substituants alkyle en C₁-C₄, alcoxy en C₁-C₄, acyle en C₂-C₄, acyloxy en C₂-C₄, phénoxy, dialkylamino en C₂-C₈, halogéno, nitro et/ou cyano.

2. Procédé de préparation de chlorures de chlorocarbonyle selon la revendication 1, **caractérisé en ce qu'**on utilise la N,N-diméthyléthylène-urée, la N,N'-diméthylpropylène-urée, la N,N'-tétrabutylurée, la N,N'-tétraméthylthiourée, la N-chlorométhyl-N'-cyanométhylpropylène-urée, la N-méthyl-N'-éthylpropylène-urée, la 1,3-diméthyl-1,3-dihydrobenzimidazole-2-one, la 1-méthyl-3-phénylimidazolidine-2,4,5-trione, la 1,3,4,6-tétraméthyl-1,3,4,6-tétrahydro-imidazo[4,5-D]imidazole-2,5-dione et la 4-méthoxy-1-méthyl-3-phénylimidazoiidine-2-thione.

3. Procédé de préparation de chlorures de chlorocarbonyle selon les revendications 1 ou 2, **caractérisé en ce que** le composé de l'urée est utilisé selon un rapport en moles à la lactone II de 0,001:1 à 0,2:1.

4. Procédé de préparation de chlorures de chlorocarbonyle selon les revendications 1 à 3, **caractérisé en ce qu'**on utilise en tant qu'agent de chloration le phosgène, le chlorure de thionyle, le chlorure d'oxalyle ou le trichlorure de phosphore.

5. Procédé de préparation de chlorures de chlorocarbonyle selon les revendications 1 à 4, **caractérisé en ce que** l'agent de chloration est utilisé selon un rapport en moles à la lactone II de 0,5:1 à 50:1.

6. Procédé de préparation de chlorures de chlorocarbonyle selon les revendications 1 à 5, **caractérisé en ce qu'**on procède à la réaction en présence d'acide chlorhydrique.

7. Procédé de préparation de chlorures de chlorocarbonyle selon les revendications 1 à 6, **caractérisé en ce qu'**on met en oeuvre la réaction à des températures de -20 à 180°C.
